# EUROPEAN PATENT SPECIFICATION

(11) **EP 0 424 836 B1**
(45) Date of publication and mention of the grant of the patent: **15.03.1995**
(21) Application number: 90120195.4
(22) Date of filing: 22.10.1990
(51) Int. Cl.: C07C 265/14, C07C 263/10

(54) **Preparation process of aliphatic isocyanates**
Verfahren zur Herstellung von aliphatischen Isozyanaten
Procédé pour la préparation d'isocyanates aliphatiques

(30) Priority: 23.10.1989 JP 273811/89; 24.10.1989 JP 275047/89
(43) Date of publication of application: 02.05.1991
(73) Proprietor: MITSUI TOATSU CHEMICALS, Inc., Chiyoda-Ku Tokyo 100 (JP)
(72) Inventor: Nagata, Teruyuki, Ohmuta-shi, Fukuoka-ken 836 (JP); Wada, Masaru, Ohmuta-shi, Fukuoka-ken 837 (JP); Mizuta, Hideki, Ohmuta-shi, Fukuoka-ken 837 (JP)
(74) Representative: VOSSIUS & PARTNER

(56) References cited:
- EP-A- 0 384 463
- FR-A- 1 071 628
- FR-A- 1 071 629
- GB-A- 1 050 555
- GB-A- 1 086 782
- US-A- 3 631 198

## Description

The present invention relates to a process for the preparation of isocyanates, particularly a process for preparation of aliphatic isocyanates by phosgenating an hydrochloride of an aliphatic polyamine.

The isocyanate obtained by the invention is a very useful compound as a raw material for polyurethane-based materials, polyurea-based materials and polyisocyaurate-based materials in chemical, resin and paint industries.

The process for preparing aliphatic isocyanates by phosgenating aliphatic amines or a salt thereof has already been known and following processes have been proposed.
(1) A process for carrying out phosgenation of aliphatic amine or its hydrochloride at temperature of 120 to 180 °C in a weight ratio of solvent/raw material amine of 18/1 to 30/1 [GB-A-1,086,782].
(2) A process for reacting phosgene with a mixture of an aliphatic triamino compound and hexamethylenetriamine having a ratio of 95:5 to 90:10 by weight [JP-60-233,044(1985)].

Other processes which have also been proposed are a continuous high-temperature phosgenation process (JP-A-55-88,451(1980)), increased pressure phosgenation process [US-A-2,642,449] and vapor phase phosgenation process (JP-A-63-280,050(1988)).

USP 3, 631, 198 has disclosed a process for preparing 1,5-diisocyanato-2 methylpentane by reacting 1,5-diamino-2-methyl pentane or its hydrochloride or carbamate or carbamylchloride salts thereof with phosgene in the presence of a lower alkylester of phthalic acid as reaction solvent.

On the other hand, GB 1, 050, 555 has disclosed a process for preparing an organic isocyanate by phosgenation of primary amine hydrochloride containing a surface-active substances or its slurry in the inert organic liquid diluents.

Processes to prepare isocyanates by reacting primary amines with phosgene in an inert solvent have been known. When the primary amines are aromatic amines, the aromatic amine can be converted with comparative ease to a high-purity aromatic isocyanate by passing phosgene gas through a suspension of free base or hydrochloride of the aromatic amine in the solvent. In the case of aliphatic amines, however, the reaction with phosgene is generally slow as compared with the reaction of aromatic amine with phosgene, and forms, as well known in the art, chloroderivatives as by-products due to a deamination reaction.

The chlorinated impurity is usually formed in an amount of 3 to 10 % by weight and sometimes goes up to 20 % by weight. Hence the yield of the desired product undergoes a corresponding decrease.

Formation of the chlorinated impurity is primarily observed in the syntheses of aliphatic isocyanates and mostly not found in the preparation of aromatic isocyanates.

When aliphatic isocyanates containing the chlorinated impurity are used for a polyurethane-based material, the chlorinated impurity affects the reaction of isocyanate group with active hydrogen containing compounds. That is, the chlorinated impurity inhibits the reaction, accelerates gelation of the prepolymer and further exerts an adverse effect on the properties of the resulting polyurethane resin.

No difference is generally observed between the properties of the chlorinated impurity and corresponding isocyanate except that the boiling point of the chlorinated impurity is generally from 5 to 20 °C lower than that of the corresponding isocyanate. Specific procedures are hence required for removing the impurity and separating a high-purity aliphatic isocyanate.

Consequently, the above-mentioned processes have been proposed in order to restrict the formation of impurity as low as possible in the phosgenation step. These processes in the prior art, however, have been disadvantageous in that use of a large amount of the solvent leads to low volume efficiency and very poor economy, and that extensive purification equipment is required for removing the impurity from the desired isocyanate. As a result, conventional processes have been unsatisfactory in view of industrial production.

One object of the present invention is to provide a process for the preparation of an aliphatic polyisocyanate having a low content of the chlorinated impurity by phosgenating an hydrochloride of an aliphatic polyamine.

Another object of the present invention is to provide a process for preparing an aliphatic polyisocyanate with good volume efficiency in the reaction.

These objects could be accomplished on the basis of the finding that selection of an ester as a reaction solvent can surprisingly prepare a desired isocyanate having a very low content of the chlorinated impurity with good volume efficiency in the reaction, and also that an aliphatic polyisocyanate containing a further reduced quantity of the impurity can be prepared by using the ester as a reaction solvent and conducting phosgenation after converting the raw material amine to the hydrochloride with hydrogen chloride gas at a temperature of 100 to 160°C.

That is, one aspect of the present invention is a process for the preparation of an aliphatic polyisocyanate by reacting an hydrochloride of an aliphatic polyamine with phosgene in the presence of an ester as a reaction solvent.

Another aspect of the invention is a process for the preparation of an aliphatic polyisocyanate comprising using an ester as a reaction solvent and conducting phosgenation after converting the raw material amine to the hydrochloride with hydrogen chloride gas at a temperature of 100 to 160°C.

According to the process of the invention, an aliphatic polyisocyanate having an extremely low content of chlorinated impurity can be obtained, post treatment steps such as purification by distillation can be simplified and loss of product due to heat deterioration in the post treatment steps can be decreased. Therefore, the process of the present invention is valuable as an industrial production process.

Exemplary polyamines used in the present invention include straight chain aliphatic diamines such as pentamethylenediamine, hexamethylenediamine, heptamethylenediamine, octamethylenediamine, nonamethylenediamine and decamethylenediamine; branched chain aliphatic polyamine such as 2,2′-dimethyl-1,3-propanediamine, 2-methyl-1,5-pentanediamine, 2,5-dimethyl-2,5-hexanediamine and 4-aminomethyloctane-1,8-diamine; and amino-acid based polyamines such as lysine methyl ester, lysine aminoethyl ester and cystine dimethyl ester.

The reaction solvent used in the process of this invention comprises an ester. Various kinds of esters can be used, and fatty acid alkyl esters and aromatic carboxylic acid esters are preferred in particular. Exemplary fatty acid alky esters include amyl formate, n-butyl acetate, isobutyl acetate, n-amyl acetate, isoamyl acetate, methylisoamyl acetate, methoxybutyl acetate, sec-hexyl acetate, 2-ethylbutyl acetate, 2-ethylhexyl acetate, cyclohexyl acetate, methylcyclohexyl acetate, benzyl acetate, ethyl propionate, n-butyl propionate, isoamyl propionate, ethyl acetate, butyl stearate, butyl lactate and amyl lactate. Aromatic carboxylic acid esters include, for example, methyl salicylate, dimethyl phthalate and methyl benzoate. More preferred esters are aliphatic esters having a boiling point of 120 to 170 °C under the atmosphoric pressure. The use of these esters is preferred in view of preventing the decomposition of isocyanates due to over-heating. These solvents can be used singly or in combination.

The amount of the solvent used in the process of this invention is preferably at a weight ratio of solvent/raw material amine in the range of 8/1 to 16/1.

When the weight ratio is less than 8/1, a large amount of amine hydrochloride is deposited and the reaction mixture becomes difficult ot stir. On the other hand, a weight ratio exceeding 16/1 has little effect on the acceleration of the reaction rate and requires larger amount of the solvent. Hence thermal efficiency in the concentration step is reduced and volume efficiency becomes industrially unfavorable.

In order to react the above aliphatic polyamine in the ester solvent mentioned above, hydrogen chloride is blown through the liquid suspending the aliphatic polyamine in the ester solvent to convert the aliphatic polyamine to hydrochloride thereof, more preferably to convert the aliphatic polyamine to hydrochloride thereof in a temperature range of 100 to 160 °C, and then excessive amount of phosgene corresponding to 2 to 10 times the mole of amino groupo is blown through the liquid to conduct phosgenation at a temperature of 100 to 170°C.

In the process for reacting phosgene after converting the aliphatic polyamine to the hydrochloride thereof with hydrogen chloride gas, no particular limitation is imposed on the temperature for converting the aliphatic polyamine to hydrochloride thereof. Particularly preferred temperatur is in the range of 100 to 160 °C. In order to obtain an aliphatic polyisocyanate having an extremely low content of the chlorinated impurity, the temperature for preparing the aliphatic polyamine hydrochloride is preferably in the above-mentioned range. When the temperature is lower than 100 °C, the amount of chlorinated impurity formed as a by-product is liable to increase. Although the hydrochloride is prepared at a temperature exceeding 160 °C, further improvement in the inhibiting effect for the chlorinated impurity cannot be found and additionally, hydrochloride preparation under the conditions of strong acidity by hydrochloric acid and high temperature is unpractical because problems on reactor material occur. The excellent results obtained in the temperature of 100 to 160 °C is assumed to be dependent upon the solubility and particle size of the aliphatic polyamine hydrochloride.

In addition,the phosgenation process of the aliphatic polyamine hydrochloride may be carried out by using the suspension prepared by suspending separately the aliphatic polyamine hydrochloride in an ester and conducting the phosgenation at a temperature of 100 to 170°C.

In the phosgenation process of an aliphatic polyamine hydrochloride obtained by using hydrogen chloride gas in the temperature range of 100 to 160 °C, an inhibiting effect for the formation of the chlorinated impurity can be found by using the esters as a solvent.

The amount of phosgene used is the same as in the case of conventional phosgenation of amine compounds, that is, an excessive amount of 2 to 10 times the mole of amino group. Preferred phosgenation temperature is in the range of 100 to 170 °C. Since thermal stability of aliphatic isocyanates is generally poor at high temperatures, phosgenation for a long time tends to cause deterioration of formed isocyanate, increase in tar content and reduction of yield. On the other hand, when the reaction temperature is too low, the reaction rate is very low and unpractical, even though the reaction proceeds.

The process of the invention can be carried out under atmospheric pressure. In order to increase the reaction rate and inhibit formation of the chlorinated impurity, the process of the invention can also be conducted under increased pressure.

Typical preferred embodiments of the process of the present invention be illustrated hereinafter.

To a reaction vessel equipped with a reflux condenser, thermometer, phosgene (or hydrogen chloride gas) inlet tube and a stirrer, raw material aliphatic amine and a reaction solvent are charged. In the case of phosgenating the aliphatic amine after converting it to the hydrochloride, the prescribed amount of hydrogen chloride gas is blown through the aliphatic amine while maintaining the prescribed range of temperature, preferably from 100 to 160 °C and then phosgen is blown through the aliphatic amine hydrochloride thus formed to carry out phosgenation.

After completing the reaction, unreacted phosgene and hydrogen chloride are purged with nitrogen and the solvent is removed. The residue is distilled to obtain pure aliphatic isocyanate.

The present invention will hereinafter be illustrated in detail by way of examples and comparative examples.

### Example 1

To a 1 ℓ reaction flask equipped with a reflux condenser, thermometer, phosgene or hydrogen chloride gas inlet tube and a stirrer, 46.5 g (0.4 mole) of raw material hexamethylenediamine (hereinafter abbreviated as HDA) and 613.5 g of n-hexyl acetate as a solvent were charged.

Under stirring and cooling, 35 g of hydrogen chloride gas was blown over an hour while rising the internal temperature to 60 °C. The resulting mixture was then heated to 155 °C and then phosgene was blown at a rate of 29.4 g/hr. The reaction was conducted for 15 hours while maintaining the temperature at 155 to 160 °C.

After finishing the reaction, unreacted phosgene and hydrogen chloride were purged with nitrogen and solvent was removed. The residue was distilled under reduced pressure of 1 to 2 mm Hg (133.3 to 266.6 Pa) to obtain 80.7 g of the product.

The product was hexamethylene diisocyanate (hereinafter abbreviated as HDI) containing 0.2 % by weight of 6-chlorohexane diisocyanate (hereinafter abbreviated as CHI). The yield of HDI was 90.0 % on the purity basis. Amount of CHI formed was 0.19 % by mole of HDA.

### Example 2

HDA and n-hexyl acetate were charged to the reaction vessel by the same procedures as described in Example 1 and cooled to 5 °C. Successively, phosgene was blown at a rate of 30 g/hr over 3 hours while maintaining the internal temperature at 0 to 5 °C.

The rate of phosgene blowing was maintained at 29.4 g/hr and the internal temperature was raised to 155 °C. Successively, the reaction was conducted for 12 hours while maintaining the internal temperature at 155 to 160 °C. After finishing the reaction, the reaction mixture was post-treated as described in Example 1 to obtain 60.6 g of HDI (88.9 % yield on purity basis). The content of CHI in HDI was 0.3 % by weight, which corresponded to 0.82 % by mole of HDA.

### Comparative Example 1

### (Trace of Example 10 in BP 1,086,782)

To the same reaction vessel as described in Example 1, 29.0 g of HDA and 735.0 g of chlorobenzene were charged and the reaction was carried out at 125 to 126 °C. Other procedures conducted in reaction and post-treatment were the same as described in Example 1. Thus 38.1 g of HDI was obtained. The yield was 87.9 % on purity basis. The content of CHI was 1.1 % by weight, which corresponded to 1.04 % by mole of HDA.

### Example 3 and Comparative Examples 2-4

The same procedures as described in Example 1 were carried out in the reaction and post treatment except that solvents illustrated in Table 1 were used in order to investigate the effect of solvents on the formation of CHI.

**Table 1**

| | Solvent | HDI (mol %/HDA) | CHI (mol %/HDA) |
|---|---|---|---|
| Example 3 | Cychlohexyl Acetate | 89.2 | 0.30 |
| Comp. Ex. 2 | ODCB | 75.9 | 3.35 |
| Comp. Ex. 3 | Mesitylene | 74.2 | 3.67 |
| Comp. Ex. 4 | MDI | 52.4 | 16.62 |
| (Note) ODCB : o-Dichlorobenzene DMI : 1,3-Dimethylimidazolidinone | | | |

### Example 4 and Comparative Examples 5-7

The same procedures as described in Example 1 and comparative Examples 2 to 4 were carried out in the reaction and post-treatment except that 46.5 g (0.4 mole) of 2-methyl-1,5-pentanediamine (hereinafter abbreviated as MPDA) as a raw material and the reaction was carried out for 18 hours. Investigation was conducted on the effect of solvent upon the formation of chlorinated impurities, that is, 5-chloro-2-methylpentane isocyanate and 5-chloro-4-methylpentane isocyanate (hereinafter abbreviated as CMPI on the whole). The yield of 2-methylpentane diisocyanate (hereinafter abbreviated as MPDI) and the amount of CMPI formed are illustrated in Table 2.

**Table 2**

| | Solvent | MPDI (mol %/MPDA) | CMPI (mol %/MPDA) |
|---|---|---|---|
| Example 4 | n-Hexyl Acetate | 89.8 | 0.19 |
| Comp. Ex. 5 | ODCB | 77.2 | 3.17 |
| Comp. Ex. 6 | Mesitylene | 75.3 | 3.42 |
| Comp. Ex. 7 | MDI | 51.7 | 18.38 |
| (Note) ODCB : o-Dichlorobenzene DMI : 1,3-Dimethylimidazolidinone | | | |

### Example 5

To the same reaction vessel as described in Example 1, 44.2 g (0.15 mole) of lysine β-aminoehtyl ester trihydrochloride (hereinafter abbreviated as LAET) and 618.8 of amyl acetate were charged, heated to 135 °C with stirring, introduced phosgen at a rate of 14.8 g/hr, and reacted for 15 hours at 135 to 140 °C

After finishing the reaction, post-treatment was conducted by the same procedures as described in Example 1 except that distillation under reduced pressure was conducted at 0.1 mm Hg (13.33 Pa). Thus, 34.5 g of lysineisocyanato-β-isocyanatoethyl ester (hereinafter abbreviated as LTI) was obtained. The yield was 85.5 % on purity basis. The chlorinated impurity, that is, lysin isocyanate β-chloroethyl ester and 2-isocyanatoethyl-2-isocyanato-6-chlorohexanate (hereinafter abbreviated as CLI on the whole) were contained in a total amount of 0.8 % by weight. The amount of CLI formed was 0.71 % by mole of LAET.

### Comparative Examples 8-10

The same procedures as described in Example 5 were carried out except that solvents were used as illustrated in Table 3 to investigate effect of solvent.

Results on Example 5 and Comparative Examples 8-10 are illustrated in Table 3.

**Table 3**

| | Solvent | LTI (mol %/LAET) | CLI (mol %/LAET) |
|---|---|---|---|
| Example 5 | Amyl Acetate | 85.5 | 0.71 |
| Comp. Ex. 8 | ODCB | 81.8 | 5.28 |
| Comp. Ex. 9 | Mesitylene | 78.1 | 5.69 |
| Comp. Ex. 10 | MDI | 56.4 | 19.80 |
| (Note) ODCB : o-Dichlorobenzene DMI : 1,3-Dimethylimidazolidinone | | | |

### Example 6

To the same reactor as described in Example 1, 46.5 g (0.4 mole) of raw material hexamethylene diamine (hereinafter abbreviated as HDA) and 613.5 g of n-hexyl acetate as a solvent were charged. The mixture was heated to 100 °C with stirring and 35 g of hydrogen chloride gas was blown over an hour while maintaining the internal temperature at 135 to 140°C. Successively, phosgene was blown at a rate of 29.4 g/hour and the reaction was continued for 15 hours at a temperature of 155 to 160 °C.

After finishing the reaction, unreacted phosgene and hydrogen chloride were purged with nitrogen and the solvent was removed. The residue was distilled under reduced pressure of 1-2 mm Hg to obtain 80.7 g of hexamethylene diisocyanate (hereinafter abbreviated as HDI). The yield was 90.0 % an purity basis. The content of 6-chlorohexane diisocyanate (hereinafter abbreviated as CHI) was 0.1 % by weight, which corresponds to 0.09 mole % of HDA.

### Comparative Examples 11-12

The same procedures as described in Example 6 were carried out in the reaction and post-treatment exept that solvents and hydrochloride forming temperatures were used as illustrated in Table 4.

Results are illustrated in Table 4.

**Table 4**

| | Solvent | Hydrochloride forming temperature (°C) | HDI (mol %/HDA) | CHI (mol %/HDA) |
|---|---|---|---|---|
| Example 6 | n-Hexyl Acetate | 135-140 | 90.0 | 0.09 |
| Comp. Ex. 11 | ODCB | 35-95 | 75.9 | 3.35 |
| Comp. Ex. 12 | mesitylene | 25-60 | 74.2 | 3.67 |
| (Note) ODCB : o-Dichlorobenzene | | | | |

### Example 7 and Comparative Examples 13-14

The same procedures as described in Examples 6 were carried out in the reaction and post-treatment except that 46.5 g (0.4 mole) of 2-methyl-4,5-pentamediamine (hereinafter abbraviated as MPDA) was used and the reaction was carried out for 18 hours. Investigation was carried out on the effect of solvents upon the chlorinated impurity, that is, 5-chloro-2-methylpentane isocyanate and 5-chloro-4-methylpentane isocyanate (hereinafter abbreviated CMPI on the whole). The yield of 2-methylpentane diisocyanate (hereinafter abbreviated as MPDI) and the amount of CMPI formed were illustrated in Table 5.

**Table 5**

| | Solvent | Hydrochloride forming temperature (°C) | MPDI (mol %/MPDA) | CMDI (mol %/MPDA) |
|---|---|---|---|---|
| Example 7 | n-Hexyl acetate | 135-140 | 89.5 | 0.09 |
| Comp. Ex. 13 | ODCB | 35-95 | 74.7 | 3.46 |
| Comp. Ex. 14 | mesitylene | 25-60 | 75.3 | 3.42 |
| (Note) ODCB : o-Dichlorobenzene | | | | |

## Claims

1. A process for the preparation of an aliphatic polyisocyanate comprising
(i) converting a polyamine to a polyamine hydrochloride by reacting the polyamine with hydrogen chloride gas at a temperature of from 100 to 160°C in the presence of an ester as reaction solvent, and then
(ii) reacting the aliphatic polyamine hydrochloride with phosgene.

2. A process according to claim 1, wherein the ester is a fatty acid alkyl ester.

3. A process according to claim 1 or 2, wherein the amount of the reaction solvent is in a ratio from 8/1 to 16/1 by weight to the aliphatic polyamine.

4. A process according to any of claims 1 to 3, wherein the polyamine is hexamethylenediamine.

## Patentansprüche

1. Verfahren zur Herstellung eines aliphatischen Polyisozyanats, umfassend
(i) die Umwandlung eines Polyamins in ein Polyaminhydrochlorid durch Umsetzung des Polyamins mit Chlorwasserstoffgas bei einer Temperatur von 100 bis 160°C in Gegenwart eines Esters als Lösungsmittel der Umsetzung, und anschließend
(ii) die Umsetzung des aliphatischen Polyaminhydrochlorids mit Phosgen.

2. Verfahren nach Anspruch 1, in dem der Ester ein Fettsäurealkylester ist.

3. Verfahren nach Anspruch 1 oder 2, in dem die Menge des Lösungsmittels der Umsetzung in einem Gewichtsverhältnis von 8/1 bis 16/1 zum aliphatischen Polyamin steht.

4. Verfahren nach irgendeinem der Ansprüche 1 bis 3, in dem das Polyamin Hexamethylendiamin ist.

## Revendications

1. Procédé de préparation d'un polyisocyanate aliphatique, comprenant
(i) la transformation d'une polyamine en chlorhydrate de polyamine, par réaction de la polyamine avec du chlorure d'hydrogène gazeux, à une température de 100 à 160°C, en présence d'un ester comme solvant réactionnel, puis
(ii) la réaction du chlorhydrate de polyamine aliphatique avec du phosgène.

2. Procédé selon la revendication 1, dans lequel l'ester est un ester alkylique d'acide gras.

3. Procédé selon la revendication 1 ou 2, dans lequel le rapport pondéral de la quantité de solvant réactionnel à la quantité de polyamine aliphatique est de 8/1 à 16/1.

4. Procédé selon l'une quelconque des revendications 1 à 3, dans lequel la polyamine est l'hexaméthylène-diamine.
